# EUROPÄISCHE PATENTSCHRIFT

(11) **EP 0 691 846 B1**
(45) Veröffentlichungstag und Bekanntmachung des Hinweises auf die Patenterteilung: **01.08.2001**
(21) Anmeldenummer: 94911933.3
(22) Anmeldetag: 19.03.1994
(51) Int. Cl.: A61K 31/495

(54) **IMIDAZOLOCHINOXALINONE ZUR BEHANDLUNG ZENTRALNERVÖSER ERKRANKUNGEN**
IMIDAZOLO-QUINOXALINONES FOR TREATING DISEASES OF THE CENTRAL NERVOUS SYSTEM
IMIDAZO-QUINOXALINONES POUR LE TRAITEMENT DE MALADIES DU SYSTEME NERVEUX CENTRAL

(30) Priorität: 31.03.1993 DE 4310523
(43) Veröffentlichungstag der Anmeldung: 17.01.1996
(73) Patentinhaber: BASF AKTIENGESELLSCHAFT, 67056 Ludwigshafen (DE)
(72) Erfinder: TREIBER, Hans-Joerg, D-68782 Bruehl (DE); BEHL, Berthold, D-67117 Limburgerhof (DE); HOFMANN, Hans Peter, D-67117 Limburgerhof (DE)
(74) Vertreter: Grünecker, Kinkeldey, Stockmair & Schwanhäusser Anwaltssozietät
(86) Internationale Anmeldenummer: EP9400872
(87) Internationale Veröffentlichungsnummer: WO9422447

(56) Entgegenhaltungen:
- EP-A- 0 348 872
- EP-A- 0 518 530
- WO-A-91/18892
- WO-A-93/20077
- DE-A- 3 004 750
- DE-A- 3 004 751
- US-A- 5 182 386
- J. MED. CHEM., Bd.35, Nr.18, 1992 Seiten 3319 - 3324 L.A. MC QUAID ET AL. 'Synthesis and excitatory amino acid pharmacology of a series of heterocyclic-fused quinoxalinones and quinazolinones'
- J. MED. CHEM., Bd.31, Nr.6, 1988 Seiten 1098 - 1115 I.R. AGER ET AL. 'Synthesis and oral antiallergic activity of carboxylic acids derived from Imidazo(2,1.c)(1,4)benzoxazines,...'

## Beschreibung

Die vorliegende Erfindung betrifft die Verwendung von Imidazolochinoxalinonen zur Behandlung von zentralnervösen Erkrankungen.

Imidazolo-chinoxalinone der Formel I sind bereits aus der DE-OS 30 04 750 und der DE-OS 30 04 751 bekannt. Für diese Verbindungen wird eine antiallergische Wirkung angegeben.

Es ist weiter bekannt, daß bestimmte, aus der EP-OS 518 530 (≙ US 5 153 196) bekannte Imidazolo-chinoxalinone eine antagonistische Wirkung auf die Rezeptoren der exzitatorisch wirksamen Aminosäuren besitzen. Sie eignen sich daher als neuroprotektive Agentien sowie zur Behebung von neurologischen Störungen, die mit diesem Wirkungsmechanismus einhergehen. Ferner sind eine Reihe andersartig substituierter Imidazolo-chinoxalinone als den Kreislauf beeinflussende c-AMP-Phosphodiesterase-Hemmer (EP-OS 400 583 ≙ US 5 166 344) und als GABA-Rezeptor-Liganden zur Behandlung verschiedener Störungen des zentralen Nervensystems (US 5 182 386) bekannt.

Gegenstand der Erfindung ist die Verwendung von Verbindungen der Formel I 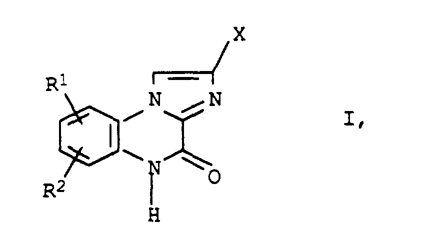 in der
- x: eine Carboxylgruppe, die in Form ihres Salzes mit einem physiologisch verträglichen Amin- oder Metallkation vorliegen kann; den Rest 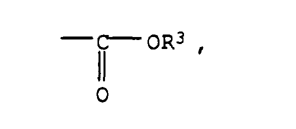 worin R³ für einen C₁₋₈-Alkyl- oder Benzylrest steht; oder X eine Hydroxymethyl-, Nitril-, Aldehyd-, Tetrazolyl-, Carbonylaminotetrazol-, Carbamoyl-, Oxim- oder C₁-C₃-Alkoximethergruppe bedeutet und
- R¹ und R²,: die gleich sind und Wasserstoff-, Chlor- oder Bromatome, darstellen,
zur Herstellung von Arzneimitteln zur Behandlung von Erkrankungen des zentralen Nervensystems.

Als Erkrankungen des zentralen Nervensystems kommen beispielsweise Epilepsie, Hirnschädigungen, Morbus Parkinson, Morbus Alzheimer, Emesis, Kopf- und Rückenmarkstrauma in Betracht. Die Wirkung der Verbindungen beruht auf deren glutamatantagonistischen Eigenschaften.

Die neue pharmakologische Wirksamkeit der Verbindungen I wurde an isoliertem Membranmaterial von Rattengroßhirnen untersucht. Hierzu wurde das Membranmaterial in Gegenwart der Verbindungen mit den radioaktiv markierten Substanzen ³H-2-Amino-3-hydroxy-5-methyl-4-isoxazolpropionsäure (³H-AMPA) und ³H-5,7-Dichlorkynurensäure behandelt, wobei sich diese an spezifische Rezeptoren (AMPA- bzw. NMDA-Rezeptor (N-Methyl-D-aspartat)) binden. Anschließend wurde durch Scintillationszählung die Radioaktivität der behandelten Membranen gemessen. Über die gebundene Radioaktivität ließen sich die Mengen an gebundener ³H-AMPA und ³H-5,7-Dichlor-kynurensäure bzw. jeweils die verdrängten Mengen dieser radioaktiv markierten Substanzen bestimmen. Die sich hieraus ergebende Dissoziationskonstante K_{I} (I = Inhibitor), welche ein Maß für die Verdrängungswirkung des erfindungsgemäßen Wirkstoffes ist, wurde durch iterative nichtlineare Regressionsanalyse mit dem Statistical Analysis System (SAS) an einem IBM-Rechner, ähnlich dem Programm "Ligand" von P.J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:
1. Bindung von ³H-2-Amino-3-hydroxy-5-methyl-4-isoxazolpropionsäure (³H-AMPA)
Für die Präparation des Membranmaterials wurden frisch entnommene Rattengroßhirne zusammen mit dem 15fachen Volumen einer Pufferlösung A aus 30 mM α,α,α-Tris-(hydroxymethyl)-methylamin-Hydrochlorid (TRIS-HC1) und 0,5 mM Ethylendiamintetraessigsäure (EDTA) - pH 7,4 - mittels eines Ultra-TURRAX-Rührers homogenisiert. Die Suspension wurde 20 Minuten bei 48 000 g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20 minütiges Zentrifugieren bei 48 000 g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.
Für den Bindungstest wurde das bei 37°C aufgetaute Proteinmaterial zweimal durch Zentrifugieren bei 48 000 g (20 Minuten) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I in 1 ml Pufferlösung B gelöst und 60 Minuten auf Eis inkubiert. Die inkubierte Lösung wurde über einen CF/B-Filter (Firma Whatman), der zuvor mindestens 2 Stunden mit einer 0,5 %igen wäßrigen Lösung von Polyethylenimin behandelt worden war, filtriert. Anschließend wurde das Filtrat mit 5 ml kalter Pufferlösung B gewaschen, um gebundene und freie ³H-AMPA voneinander zu trennen. Nach Messung der Radioaktivität der gebundenen 3H-AMPA im Membranmaterial durch Scintillationszählung wurde durch Auswertung der Verdrängungskurven mittels Regressionsanalyse der K_{I}-Wert bestimmt.
2. 3H-Glycin-Bindungsassay
Frisch entnommene Rattenhippocampi werden in 10fachen Volumen Präparationspuffer (50 mM Tris-HCl, 10 mM EDTA) mit einem Potter-Homogenisator homogenisiert. Das Homogenat wird 20 Minuten bei 48000 x g zentrifugiert. Der Überstand wird verworfen und die im Pellet erhaltenen Membranen durch Resuspendieren und Zentrifugieren bei 48000 x g (jeweils 20 Minuten) 2 x gewaschen. Die resuspendierten Membranen werden in flüssigen Stickstoff eingefroren und bei 37°C wieder aufgetaut. Nach einem erneuten Waschschritt wird die Membransuspension 15 Minuten bei 37°C im Schüttelwasserbad inkubiert. Nach 4 weiteren Waschschritten (jeweils 20 minütiges Zentrifugieren bei 48000 x g und Resuspendieren in Präparationspuffer) werden die Membranen bis zur weiteren Verwendung bei -70°C eingefroren.
Die eingefrorenen Membranen werden bei 37°C aufgetaut und 2 x durch Zentrifugation bei 48000 x g (20 Minuten) und anschließendes Resuspendieren in Bindungspuffer (50 mM Tris-HCl pH 7,4; 10 mM MgCl₂) gewaschen. Ein Inkubationsansatz enthält 0,25 mg Protein (Membranen), 25 nM ³H-Glycin (16 Ci/mMol) und die zu testenden Substanzen in insgesamt 0,5 ml Bindungspuffer. Die unspezifische Bindung wird durch Zugabe von 1 mM Glycin bestimmt. Nach 60minütiger Inkubation bei 4°C wird gebundener und freier Ligand durch Filtration über GF/B-Filter und anschließendem Waschen mit ca. 5 ml eiskaltem Bindungspuffer voneinander getrennt. Die auf den Filtern verbleibende Radioaktivität wird durch Flüssigkeitsscintillationszählung bestimmt. Aus den Verdrängungskurven werden mit Hilfe ines iterativen nichtlinearen Anpassungsprogramms oder entsprechend der Gleichung von Cheng und Prusoff die Dissoziationskonstanten berechnet.
In diesen Tests wurden mit den Verbindungen des Anspruchs 1 folgende Ergebnisse erzielt:

| | ³H-Glycin | ³H-AMPA |
|---|---|---|
| Substanz des Beispiels 1 | 110 | 250 |
| Substanz des Beispiels 1 (Ethylester) | 162 | 558 |

Die Arzneimittelzubereitungen enthalten neben den üblichen Arzneimittelhilfsstoffen eine therapeutisch wirksame Menge an Verbindung I. Sie können in verschiedenen Applikationsweisen verabreicht werden, wie peroral, parenteral, subkutan, intraperitonal und topisch. So sind Zubereitungsformen wie Tabletten, Emulsionen, Infusions- und Injektionslösungen, Pasten, Salben, Gele, Cremes, Lotionen, Puder und Sprays möglich.

Für die lokale äußere Anwendung, z.B. in Puder und Salben, können die Wirkstoffe darin in den üblichen Konzentrationen enthalten sein. In der Regel liegt die Menge bei 1 bis 50 Gew.-%

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg Wirkstoff gegeben. Die Zubereitungen können täglich in einer oder mehreren Dosierungen je nach Art und Schwere der Erkrankung verabreicht werden. Die Tagesdosis liegt in der Regel bei 0,1 bis 10 mg pro kg Körpergewicht bei oraler Gabe und bei 0,01 bis 50 mg pro kg Körpergewicht bei parenteraler Gabe.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes, Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Bleichmittel in den Applikationsformen enthalten sein.

Die neben dem Wirkstoff in der Zubereitung enthaltenen Stoffe sowie die bei der Herstellung der pharmazeutischen Zubereitung verwendeten Stoffe müssen toxikologisch unbedenklich und mit dem jeweiligen Wirkstoff verträglich sein.

Die Herstellung der Arzneimittelzubereitungen erfolgt in bekannter Weise.

### Beispiel 1

Auf einer Tablettenpresse werden in üblicher Weise Tabletten folgender Zusammensetzung gepreßt:

| | |
|---|---|
| 40 mg | 7,8-Dichlor-4,5-dihydro-4-oxo-imidazolo-[1,2-a]-chinoxalin-2-carbonsäure |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### Beispiel 2

In üblicher Weise werden Dragees folgender Zusammensetzung hergestellt:

| | |
|---|---|
| 20 mg | 7,8-Dichlor-4,5-dihydro-4-oxo-imidazolo-[1,2-a]-chinoxalin-2-carbonsäure |
| 60 mg | Kernmasse |
| 60 mg | Verzuckerungsmasse |

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Beispiel 3

10 g 7,8-Dichlor-4,5-dihydro-4-oxo-imidazolo-[1,2-a]chinoxalin-2-carbonsäure werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche

1. Verwendung von Verbindungen der Formel I 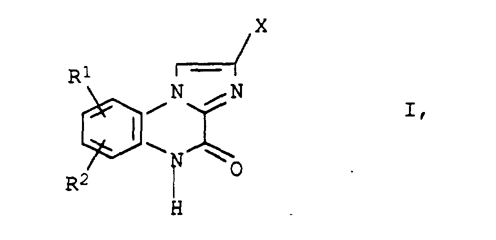 in der
x eine Carboxylgruppe, die in Form ihres Salzes mit einem physiologisch verträglichen Amin- oder Metallkation vorliegen kann; den Rest 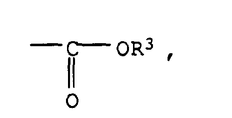 worin R³ für einen C₁₋₈-Alkyl- oder Benzylrest steht; oder X eine Hydroxymethyl-, Nitril-, Aldehyd-, Tetrazolyl-, Carbonylaminotetrazol-, Carbamoyl-, Oxim- oder C₁-C₃-Alkoximethergruppe bedeutet und
R¹ und R², die gleich sind und Wasserstoff-, Chlor- oder Bromatome, darstellen,
zur Herstellung von Arzneimitteln zur Behandlung von Erkrankungen des zentralen Nervensystems.

## Claims

1. The use of compounds of the formula I 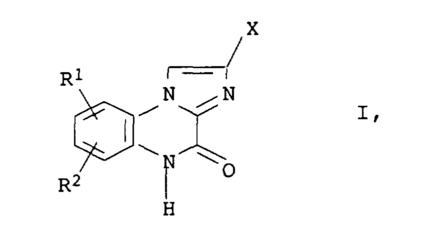 where
x is a carboxyl group which can be in the form of its salt with a physiologically tolerated amine cation or metal cation; the radical 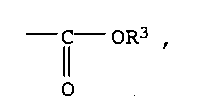 where R³ is C₁₋₈-alkyl or benzyl; or X is a hydroxymethyl, cyano, formyl, tetrazolyl, carbonylaminotetrazole, carbamoyl, oxime or C₁-C₃-alkoxime ether group and
R¹ and R² are identical and are hydrogen, chlorine or bromine atoms,
for the production of drugs for the treatment of disorders of the central nervous system.

## Revendications

1. Utilisation de composés de formule I 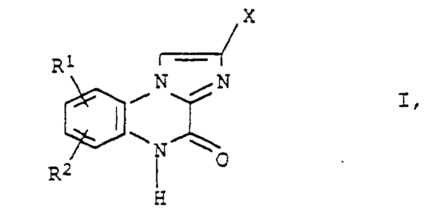 dans laquelle
X désigne un groupe carboxyle, qui peut se présenter sous la forme, de son sel avec un cation amine ou de métal physiologiquement acceptable; le reste 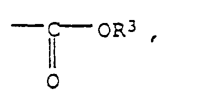 où R³ représente un reste alkyle en C₁₋₈ ou benzyle; ou X désigne un groupe hydroxyméthyle, nitrile, aldéhyde, tétrazolyle, carbonylaminotétrazole, carbamoyle, oxime ou alcoxime en C₁-C₃, et
R¹ et R², qui sont identiques et représentent des atomes d'hydrogène, de chlore ou de brome,
pour la préparation de médicaments pour le traitement d'affections du système nerveux central.
